# EUROPEAN PATENT APPLICATION

(11) **EP 4 640 665 A1**
(43) Date of publication of application: **29.10.2025**
(21) Application number: 24172649.6
(22) Date of filing: 26.04.2024
(51) Int. Cl.: C07C 51/47, C07C 53/126

(54) **METHOD OF RECOVERY AND CONCENTRATION OF MEDIUM CHAIN CARBOXYLIC ACIDS**

(71) Applicant: Politechnika Poznanska, 60-965 Poznan (PL)
(72) Inventor: Ole kowicz-Popiel, Piotr, 61-063 Pozna (PL); Brodowski, Filip, 61-324 Pozna (PL); Gutowska, Natalia, 60-480 Pozna (PL)
(74) Representative: Tyrala, Magdalena

(57) **Abstract**

The subject of the invention is a method for the recovery and concentration of medium-chain carboxylic acids, in particular hexanoic acid, from model solutions and the real fermentation broth, in which the hexanoic acid present in the multi-component model solutions and the real fermentation broth is recovered and concentrated using the pertraction technique with an additional recirculating bypass.

## Description

The subject of the invention is a method for recovering and concentrating medium-chain carboxylic acids (MCCA), in particular hexanoic acid, from process solutions, including model and raw fermentation broths, using extraction in capillary fiber membrane modules (Pertraction) with an additional recirculating bypass. Hexanoic acid (C₆H₁₂O₂) is a six-carbon, medium-chain carboxylic acid with several practical applications, including feed additive, corrosion inhibitor, substrate to produce antibiotics, raw material for the production of fragrances, flavors and biofuels (P. Candry, L. Radić, J. Favere, J. M. Carvajal-Arrayo, K. Rabaey, R. Ganigue, Water Research, 186 (2020) 116396).

Traditionally, hexanoic acid is obtained by fractional distillation of coconut, and palm oils containing less than 1% of this acid (W. de Araújo Cavalcante, R. Carrhá Leitão, T. A. Gehring, L. T. Angenent, S. Tédde Santaella, Biores. Technol, 54 (2017)106-119).

On the other hand, one of the main alternative, environmentally friendly methods of obtaining hexanoic acid is the anaerobic fermentation of waste carbon sources using open cultures (J. Chwalkowska, A. Duber, R. Zagrodnik, F. Walkiewicz, M. Lyzyk, P. Oleskowicz-Popiel, Biores. Technol. 279 (2019) 74-83). Many literature reports indicate the advantage of anaerobic biosynthesis of hexanoic acid using open cultures due to the possibility of using complex waste substrates such as acid whey or food waste, the organic fraction of municipal waste, sewage treatment plant sludge, and food silage (J. Chwalkowska, A. Duber, R. Zagrodnik, F. Walkiewicz, M. Lyzyk, P. Oleskowicz-Popiel, Biores. Technol. 279 (2019) 74-83; S. G. Arhin, A. Cesaro, F. Di Capua, G. Esposito, J. Environ. Manag., 348, 2023, 119379; J. Huang, K. Chen, X. Xia, H. Zhu, Chemosphere, 335, 2023, 139049). However, one of the main process limitations affecting the still unsatisfactory efficiency of hexanoic acid biosynthesis is the cellular toxicity of MCCA, resulting from the accumulation of undissociated forms of these acids in the bioreactor (J. Xu, B, Bian, L. T. Angenent, P. E. Saikaly, Front. Bioeng. Biotechnol., 9 (2021) 726945). Therefore, it is necessary to integrate the bioreactor with a separation unit, enabling the continuous recovery of hexanoic acid (M. T. Agler, C. M. Spirito, J. G. Usack, J. J. Werner, L. T. Angenent, Energy Environ. Sci., 5, 2012, 8189; US 2023/0332083 A1).

However, the recovery of carboxylic acids from fermentation broths is still difficult due to the relatively low concentrations of the main metabolites and the complex physicochemical structure of the separated solutions (S. A. Aktij, A. Zirehpour, A. Mollahosseini, M. J. Taherzadeh, A. Rahimpour, J. Ind. Eng. Chem., 81, 2020, 24-40; M. Braune, B. Yuan, H. Sträuber, S. Ch. McDowall, R. Nitzsche, A. Gröngröft, Front. Bioeng. Biotechnol., 9, 2021, 725578). Consequently, the cost of separation, concentration, and purification of the main metabolite obtained by microbiological conversion may constitute 50 - 80% of the total production cost. (M. Szczygielda, M. Krajewska, L. Zheng, L. D. Nghiem, K. Prochaska, J. Membr. Sci., 637, 2021, 119593).

The main separation methods of MCCA, including hexanoic acid, from fermentation broths include:
- electrodialysis (ED) (P. A. Hernandez, M. Zhou, I. Vassiliev, S. Freguia, Y. Zhang, J. Keller, P. Ledezma, B. Virdis, ACS Omega, 6, 2021, 7841-7850; J. Xu, J. L. Guzman, L. T. Angenent, Environ. Sci. Technol., 55, 2021, 634-644; L. Selder, R. Turunen, K. V. Vuoristo, J. Uusitalo, A-P. Zeng, Biores. Technol., 16, 2021, 100828),
- electrodialysis with bipolar membrane (EDBM) (W. Zhao, H. Jiang, W. Dong, Q. Liang, B. Yan, Y. Zhang, Biores. Technol., 399, 2024, 130647),
- reactive extraction (S. Mukherjee, B. Munshi, Chem. Eng. Proc. Proc. Int., 153, 2020, 107926),
- liquid-liquid extraction in capillary fiber membrane modules (pertraction) (T. A. Gehring, W. A. Cavalcante, A. S. Colares, L. T. Angenent, S. T. Santaella, M. Liibken, R. C. Leitão, J. Chem. Technol. Biotechnol., 95, 2020, 3105-3116)
- and combined systems type pertraction-electrolysis (J. Xu, J. J. L. Guzman, S. J. Andersen, K. Rabaey, L. T. Angenent, Chem. Commu., 51, 2015, 6847-6850).

Among these methods, pertraction is considered an essential separation process due to the low energy consumption needed for pumping working media and selective extraction of acids, especially those with a longer carbon chain (J. Xu, L. T. Angenent, P. E. Saikaly, Front. Bioeng. Biotechnol., 9, 2021, 726946). In the pertraction process, phase contact occurs through a hydrophobic porous membrane with a developed surface (K. Prochaska, 2013, ISBN 978-83-7775-242-5; S. Schlosser, R. Kertész. J. Marták, Sep. Purif. Technol., 41, 2005, 237-266). Typically, the aqueous phase is introduced into the capillary fibers, while the organic phase, which acts as a carrier of the extracted substance, is introduced into the jacket space. (ISBN 978-83-7775-242-5). In the case of MCCA recovery, the most frequently used organic phase is mineral oil with the addition of 3% trioctylphosphine oxide (TOPO) acting as a solvating extractant.

In the pertraction process, re-extraction takes place in the second membrane column, in which the aqueous phase introduced into the capillaries is played by an alkaline solution (e.g. NaOH or sodium tetraborate solution). The driving force of the process causing the migration of undissociated forms of hexanoic acid through the membrane is the difference in the chemical activity of ions on both sides of the membrane caused by the pH gradient (~5.0 - 9.0) (J. Xu, B, Bian, L. T. Angenent, P. E. Saikaly, Front. Bioeng. Biotechnol., 9 (2021) 726945). Significantly, before the electrodialysis or pertraction process, it is usually necessary to pre-clarify the fermentation broth from macromolecular impurities using pressure membrane processes such as micro- (MF) or ultrafiltration (UF) (J. M. Carvajal-Arroyo, S. J. Andersen, R. Ganigué, R. A. Rozendal, L. T. Angenent, K. Rabaey, Chem. Eng. J. 416 (2021) 127886).

Although pertraction is considered the best-studied method for separating and recovering MCCA from fermentation broths, the stream of the transferred substance is still limited, especially in continuous fermentation-separation processes. The main factor limiting the continuous MCCA extraction process is the narrow working pH range of the initial solution moved to pertraction (5.0 - 5.5) associated with favourable conditions for MCCA biosynthesis (C. M. Spirito, A. M. Marzili, L. T. Angenent, Environ. Sci. Technol., 52, 2018, 13438-1344). The pKa value of hexanoic acid is 4.88, which means that in the considered pH range, less than 50% of hexanoic acid occurs in an undissociated form capable of selective migration through the porous membrane towards the organic solvent (J. Xu, J. J. L. Guzman, S. J. Andersen, K. Rabaey, L. T. Angenent, Chem. Commu., 51, 2015, 6847-6850).

Moreover, literature reports indicate that in the case of food waste fermentation, the optimal pH value of the fermentation solution exceeds 5.5 (Q. Wang, G. Zhang, X. Wang, W. Fang, P. Zhang, N. Yang, Y. Wu, W. Ma, C. Fu, J. Clean, Prod., 368, 2022, 133220; S. G. Arhin, A. Cesaro, F. Di Capua, G. Esposito, J. Envir. Mana., 348, 2023, 119379).

If the hexanoic acid biosynthesis process is carried out at elevated pH > 5.5, this metabolite occurs in a completely dissociated form, which makes its simultaneous production and recovery in the continuous pertraction process impossible without prior acidification of the separated solution. Kucek et al. (L. A. Kucek, J. Xu, M. Nguyen, L. T. Angenent, Front. Microbiol. 7 (2016) 1892) indicate the possibility of improving the efficiency of MCCA pertraction by increasing the recirculation flow rate of the fermentation broth, which reduces the resistance to MCCA transfer through the membrane. However, controlling the flow rate is possible if the fermentation broth is directly moved to the extraction column. M. Carvajal-Arroyo et al. (J. M. Carvajal-Arroyo, S. J. Andersen, R. Ganigué, R. A. Rozendal, L. T. Angenent, K. Rabaey, Chem. Eng. J. 416 (2021) 127886) indicate that when using a combined ultrafiltration-pertraction system allowing for simultaneous pre-treatment of fermentation broth containing solid particles, the control of the recirculation of the working medium is limited due to the low flux of permeate (36 L·m⁻²·h⁻¹⁾ obtained after UF. In this study, the value of the mass flux of hexanoic acid was 6.6 ± 2.4 g·m⁻²day⁻¹.

During research and development work, it was established that improving the efficiency of recovery and concentration of hexanoic acid in the pertraction process is possible by using an additional recirculating circuit (bypass) allowing for additional control of the flow rate and pH control of the separated solution directed to the extraction module, regardless of the pH value solution in the bioreactor. It should be noted that there have been no reports indicating the use of the extraction process in capillary fiber membrane modules (Pertraction) with an additional recirculating bypass for the recovery and concentration of medium-chain carboxylic acids (MCCA), in particular hexanoic acid, from process liquids, including model and real fermentation solutions.

The essence of the invention is a method for recovering and concentrating medium-chain carboxylic acids, in particular hexanoic acid, from process liquids, including model and real fermentation broths, using extraction in capillary fiber membrane modules (Pertraction) with an additional recirculating bypass. By using an additional recirculating bypass, it is possible to improve the efficiency of hexanoic acid and longer MCCA recovery thanks to additional flow rate control and pH control of the solution directed to the capillary extraction module, regardless of the pH value in the bioreactor. In the method according to the invention, a multi-component model solution containing: hexanoic acid, valeric acid, butyric acid, propionic acid, acetic acid with a pH of 5.5 adjusted by the addition of sodium hydroxide, as well as the fermentation broth containing: hexanoic acid, heptanoic acid, caprylic acid, valeric acid, butyric acid, propionic acid, acetic acid with a pH ranging from 5 to 5.5 adjusted by the addition of sodium hydroxide, MCCA, and in particular hexanoic acid, is recovered and concentrated continuously using the pertraction technique, using two membrane modules equipped with in porous, hydrophobic capillary membranes with an active surface of each membrane equal to 0.035 m² under specific process conditions:
- use of a model or real fermentation solution with an initial volume ranging from 0.5 to 1 dm3 and a pH ranging from 5 to 5.5 (preferably 5) as a feed solution, recirculated in a closed system at a flow rate of 0.1 L/h,
- use of a 3% solution of trioctylphosphine oxide (TOPO) in mineral oil with an initial volume of 0.4 L as an extracting solution of an organic solvent, recirculated in a closed system at a flow rate of 19 L/h,
- use of an aqueous solution of sodium tetraborate (Borax) with a concentration of 14 g/L and an initial volume ranging from 0.25 to 0.8 L (preferably 0.8 L) and a constant pH value ranging from 8.0 to 9.0 (preferably 9) automatically regulated by the addition of 3M sodium hydroxide solution as a re-extracting alkaline solution, recirculated in a closed system at a flow rate of 6 L/h.

Moreover, during the process, the separated solution is directed to the recirculation bypass filled with a multi-component model solution with a pH of 4.5 - 5.5 adjusted by the addition of sodium hydroxide, and then to the interior of the capillary fibers of the first membrane module and recirculated in a closed system for 72 h at a constant flow rate in the recirculating bypass in the range from 0.6 to 19 L/h, preferably 6 L/h, while a 3% solution of trioctylphosphine oxide in mineral oil is directed to the jacket space of both membrane modules and recirculated in a closed system for 72 h at a constant flow rate ranging from 0.6 to 19 L/h, preferably 6 L/h, moreover, an aqueous solution of sodium tetraborate is introduced into the capillary fibers of the second membrane module and recirculated in a closed system also for 72 h at a constant flow rate in the range from 0.6 to 19 L/h, preferably 6 L/h.

It is preferred that the real fermentation broth is pre-treated to remove biomass and insoluble solids by centrifugation and microfiltration before undergoing separation and concentration by pertraction.

By using the recovery and concentration method according to the invention, the following technical and utility effects were achieved:
- effective and selective recovery of MCCA, especially hexanoic acid, from model solutions and real fermentation broth,
- possibility of simultaneous biosynthesis of MCCA and their recovery continuously, regardless of the pH value in the bioreactor,
- improving the efficiency of the hexanoic acid biosynthesis process by continuously removing the cytotoxic product,
- possibility of controlling the flow rate of the solution separated in the additional recirculating bypass,
- possibility of environmentally friendly, energy-saving concentration of MCCA, especially hexanoic acid, from model solutions and real fermentation broth.

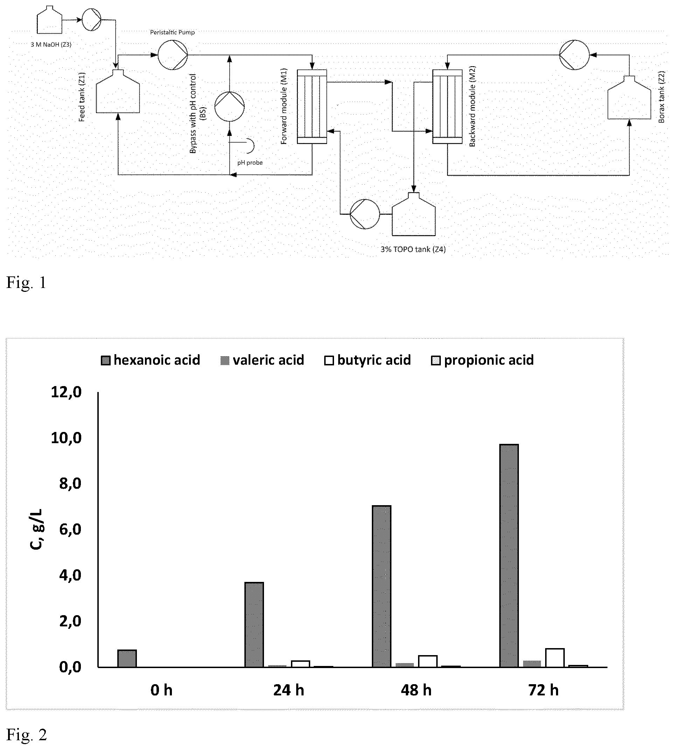

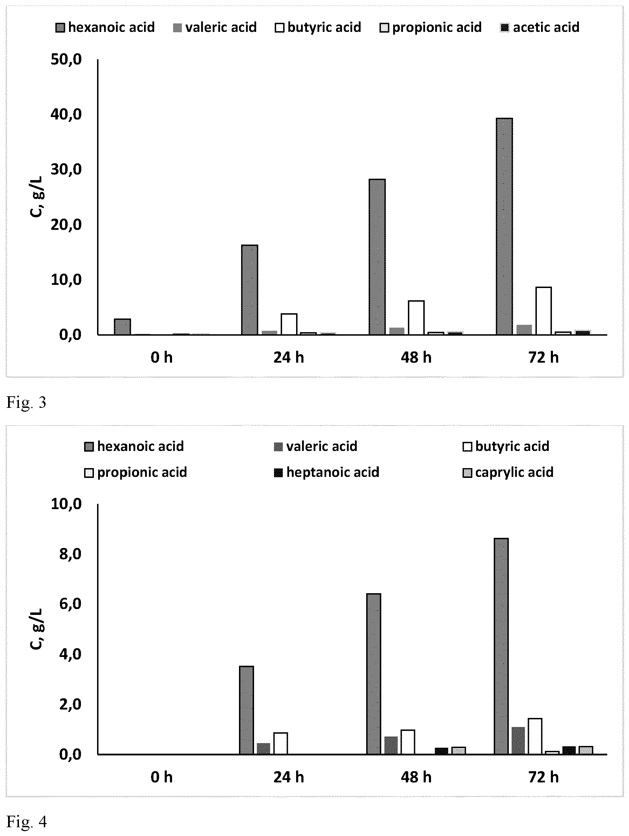

The method according to the invention in an exemplary embodiment is documented in the drawing, where Fig. 1 shows a schematic diagram of the pertraction system with an additional recirculation bypass; Fig. 2: change in the concentration of carboxylic acids in an alkaline solution of sodium tetraborate with an initial volume of 0.25 L and a constant pH value of 8.0 during the pertraction process of a multi-component model solution containing: hexanoic acid, valeric acid, butyric acid, propionic acid, acetic acid and pH of 5.5 regulated by the addition of sodium hydroxide and using additional recirculation carried out at a constant feed flow rate of 1 L/h without automatic pH correction in the recirculating bypass; Fig. 3: change in the concentration of carboxylic acids in an alkaline solution of sodium tetraborate with an initial volume of 0.25 L and a constant pH value of 9.0 during the pertraction process of a multi-component model solution containing: hexanoic acid, valeric acid, butyric acid, propionic acid, acetic acid and pH of 5.5, regulated by the addition of sodium hydroxide and using additional recirculation carried out at a constant feed flow rate of 6 L/h and automatic pH correction in the recirculating bypass to a value of 4.5; Fig. 4: change in the concentration of carboxylic acids in an alkaline sodium tetraborate solution with an initial volume of 0.8 L and a constant pH value of 8.0 during the pertraction process of the fermentation broth containing: hexanoic acid and longer MCCA, as well as valeric acid and butyric acid, propionic acid, acetic acid, and a pH of 5 regulated by the addition of sodium hydroxide and using additional recirculation carried out at a constant feed flow rate of 19 L/h without automatic pH correction in the recirculating bypass.

The method according to the invention is illustrated by the following examples:

### Example 1

Pertraction of a multi-component model solution containing: hexanoic acid at a concentration of 8 g/L, valeric acid at a concentration of 0.5 g/L, butyric acid at a concentration of 4 g/L, propionic acid at a concentration of 0.5 g/L, acetic acid 4 g/L, volume 0.5 L and pH equal to 5.5, regulated by the addition of sodium hydroxide from the Z3 tank. The separated solution was introduced into the feed tank Z1, while the solution introduced into the organic solvent tank Z4 contained a 3% solution of trioctylphosphine oxide (TOPO) in mineral oil with an initial volume of 0.4 L, while the solution introduced into the alkaline re-extractant tank Z2 was an aqueous solution of tetraborate sodium (Borax) with a concentration of 14 g/L and an initial volume of 0.25 L and a constant pH value of 8.0, automatically adjusted by the addition of 3 M sodium hydroxide solution. During the pertraction process, the model solution was continuously introduced and withdrawn into the Z1 supply tank at a constant flow rate of 0.0125 L/h. The pertraction process was carried out using two membrane modules M1 and M2 equipped with porous, hydrophobic capillary membranes with an active surface of each membrane equal to 0.035 m². The separated solution was directed to the BR recirculation bypass filled with a multi-component model solution with a pH of 5.5 adjusted by the addition of sodium hydroxide, and then to the interior of the capillary fibers of the first M1 membrane module and recirculated in a closed system for 72 h at a constant flow rate in the BR recirculation bypass equal to 1 L/h, while a solution of 3% trioctylphosphine oxide (TOPO) in mineral oil was directed to the jacket space of both membrane modules M1 and M2 and recirculated in a closed system for 72 h at a constant flow rate of 19 L/h. An aqueous solution of sodium tetraborate (Borax) was introduced into the capillary fibers of the second M2 membrane module and recirculated in a closed system for 72 h at a constant flow rate of 6 L/h. In Fig. 1 of the drawing, p refers to the peristaltic pumps supplying the system, and S pH refers to the pH probe. The change in the concentration of carboxylic acids in the alkaline sodium tetraborate solution during the pertraction process is shown in Fig. 2.

### Example 2

Pertraction of a multi-component model solution containing: hexanoic acid at a concentration of 8 g/L, valeric acid at a concentration of 0.5 g/L, butyric acid at a concentration of 4 g/L, propionic acid at a concentration of 0.5 g/L, acetic acid 4 g /L, volume 1 L and pH equal to 5.5, regulated by the addition of sodium hydroxide from the Z3 tank. The separated solution was introduced into the feed tank Z1, while the solution introduced into the organic solvent tank Z4 contained a 3% solution of trioctylphosphine oxide (TOPO) in mineral oil with an initial volume of 0.4 L, while the solution introduced into the alkaline re-extractant tank Z2 was an aqueous solution of tetraborate sodium (Borax) with a concentration of 14 g/L and an initial volume of 0.25 L and a constant pH value of 9.0, automatically adjusted by the addition of 3 M sodium hydroxide solution. During the pertraction process, the model solution was continuously introduced and withdrawn into the Z1 supply tank at a constant flow rate of 0.02 L/h. The pertraction process was carried out using two modules M1 and M2 equipped with porous, hydrophobic capillary membranes with an active surface of each membrane equal to 0.035 m². The separated solution was directed to the BR recirculation bypass filled with a multi-component model solution with a constant pH value of 4.5, adjusted automatically by the addition of 0.5 M HCl solution, and then to the interior of the capillary fibers of the first M1 membrane module and recirculated in a closed system for 72 h at a constant a flow rate in the recirculating bypass of 6 L/h, while a solution of 3% trioctylphosphine oxide (TOPO) in mineral oil was directed to the jacket space of both membrane modules and recirculated in a closed system for 72 h at a constant flow rate of 19 L/h. An aqueous solution of sodium tetraborate (Borax) was introduced into the capillary fibers of the second M2 membrane module and recirculated in a closed system for 72 h at a constant flow rate of 6 L/h. The change in the concentration of carboxylic acids in the alkaline sodium tetraborate solution during the pertraction process is shown in Fig. 3.

### Example 3

Pertraction of fermentation broth containing hexanoic acid at a concentration of 1.6 g/L, valeric acid at a concentration of 0.5 g/L, butyric acid at a concentration of 1.6 g/L, propionic acid at a concentration of 0.4 g/L, acetic acid 2.0 g/L, heptanoic acid 0.04 g/L, caprylic acid 0.028 g/L, with a volume of 0.8 L and a pH of 5 adjusted by the addition of sodium hydroxide from the Z3 tank. The separated solution was introduced into the feed tank Z1, while the solution introduced into the organic solvent tank Z4 contained a 3% solution of trioctylphosphine oxide (TOPO) in mineral oil with an initial volume of 0.4 L, while the solution introduced into the alkaline re-extractant tank Z2 was an aqueous solution of tetraborate sodium (Borax) with a concentration of 14 g/L and an initial volume of 0.8 L and a constant pH value of 8.0, automatically adjusted by the addition of 3 M sodium hydroxide solution. During the pertraction process, the fermentation solution was continuously introduced and discharged into the Z1 supply tank at a constant flow rate of 0.008 L/h. The pertraction process was carried out using two membrane modules M1 and M2 equipped with porous, hydrophobic capillary membranes with an active surface of each membrane equal to 0.035 m². The separated solution was directed to the BR recirculation bypass filled with pre-purified real fermentation broth with a pH of 5.0 adjusted by the addition of sodium hydroxide, and then to the interior of the capillary fibers of the first M1 membrane module and recirculated in a closed system for 72 h at a constant flow rate in the recirculating bypass BR of 19 L/h, while a solution of 3% trioctylphosphine oxide (TOPO) in mineral oil was directed to the jacket space of both membrane modules M1 and M2 and recirculated in a closed system for 72 h at a constant flow rate of 19 L/h. An aqueous solution of sodium tetraborate (Borax) was introduced into the capillary fibers of the second M2 membrane module and recirculated in a closed system for 72 h at a constant flow rate of 6 L/h. The change in the concentration of carboxylic acids in the alkaline sodium tetraborate solution during the pertraction process is shown in Fig. 4.

## Claims

1. Method of recovery and concentration of medium-chain carboxylic acids, in particular hexanoic acid, from process liquids, including model and real fermentation broths, using extraction in capillary fiber membrane modules, **characterized in that** from a multi-component model solution containing: hexanoic acid, valeric acid, butyric acid, propionic acid, acetic acid with a pH of 5.5 adjusted by the addition of sodium hydroxide, and the real fermentation broth containing: hexanoic acid, heptanoic acid, caprylic acid, valeric acid, butyric acid, propionic acid, acetic acid with a pH of in the range from 5 to 5.5, regulated by the addition of sodium hydroxide, medium-chain carboxylic acids, in particular hexanoic acid, are recovered and concentrated continuously using the pertraction technique, using two membrane modules (M1) and (M2) equipped with porous, hydrophobic capillary membranes with an active area of each membrane equal to 0.035 m² under specific process conditions:
• use of a model or real fermentation broth with an initial volume ranging from 0.5 to 1 L and a pH ranging from 5 to 5.5, preferably 5, as a feed solution, recirculated in a closed system at a flow rate of 0.1 L/h,
• use of a 3% solution of trioctylphosphine oxide with an initial volume of 0.4 L in mineral oil as an extracting solution of an organic solvent, recirculated in a closed system at a flow rate of 19 L/h,
• use of an aqueous solution of sodium tetraborate with a concentration of 14 g/L and an initial volume ranging from 0.25 to 0.8 L, preferably 0.8 L and a constant pH value ranging from 8.0 to 9.0, preferably 9, regulated automatically by the addition of 3 M sodium hydroxide solution as a re-extracting alkaline solution, recirculated in a closed system at a flow rate of 6 L/h, with the separated solution directed to the recirculation bypass (BR) filled with a multi-component model solution with a pH of 4.5 - 5.5 regulated by the addition of sodium hydroxide, and then into the capillary fibers of the first membrane module (M1) and recirculated in a closed system for 72 h at a constant flow rate in the recirculating bypass in the range from 0.6 to 19 L/h, preferably 6 L/h, while a solution of 3% trioctylphosphine oxide solution in mineral oil is directed to the jacket space of both membrane modules (M1) and (M2) and recirculated in a closed system for 72 h at a constant flow rate ranging from 0.6 to 19 L/h, preferably 6 L/h, moreover, an aqueous solution of sodium tetraborate is introduced into the capillary fibers of the second membrane module (M2) and recirculated in a closed system also for 72 h at a constant flow rate ranging from 0.6 to 19 L/h, preferably 6 L/h.

2. The method of claim 1, is **characterized by** purifying the real fermentation broth before the pertraction process to remove biomass and insoluble solids by centrifugation and microfiltration techniques
